# EUROPEAN PATENT APPLICATION

(11) **EP 4 741 421 A1**
(43) Date of publication of application: **13.05.2026**
(21) Application number: 24886339.1
(22) Date of filing: 01.11.2024
(51) Int. Cl.: C07K 16/18, A61P 35/00, A61K 39/00

(54) **FAB-ARM EXCHANGE-PREVENTING FC VARIANTS FROM WHICH EFFECTOR FUNCTIONS ARE REMOVED**

(30) Priority: 03.11.2023 KR 20230150769
(71) Applicant: Korea University Research and Business Foundation, Seoul 02841 (KR)
(72) Inventor: JUNG, Sang-Taek, Seoul 06217 (KR); KIM, Su-Yeon, Seoul 07211 (KR); JO, Mi-Gyeong, Seoul 02717 (KR); KYUNG, Mun-Su, Gimpo-si Gyeonggi-do 10109 (KR); KO, Woo-Hyung, Seoul 08018 (KR)
(74) Representative: Isarpatent
(86) International application number: PCT/KR2024/017028
(87) International publication number: WO 2025/095668

(57) **Abstract**

The present disclosure relates to Fab-arm exchange-preventing Fc variants having reduced effector functions due to the removal of binding affinity to FcyRs and C1q. Human antibody Fc domain variants of the present disclosure are novel variants different from conventional Fab-arm exchange-preventing variants and can overcome the Fab-arm exchange phenomenon, which is a disadvantage of IgG4, while not binding to all human FcyRs and C1q and not binding to mouse and monkey Fc7Rs, and have excellent blood half-life and thermal stability, and thus can be used for the prevention of immune cell/normal cell death (toxicity) caused by an antibody Fc region of a therapeutic antibody or Fc-fusion protein drug.

## Description

### [Technical Field]

The present disclosure relates to Fab-arm exchange-preventing Fc variants having reduced effector functions due to the removal of binding affinity to FcyRs and C1q.

### [Background Art]

Protein therapeutic agents have been widely used in clinical trials by rapidly replacing non-specific low molecular compound therapeutic agents due to very high specificity for disease targets and low side effects and toxicity. Among protein therapeutic agents which have been currently used in clinical trials, antibody therapeutics and Fc-fusion protein therapeutics fused with antibody Fc regions make up the largest part. Therapeutic antibodies are considered as one of the most effective cancer therapies by exhibiting very high target specificity compared to conventional low molecular drugs, and having not only low biotoxicity and few side effects, but also an excellent blood half-life of about 3 weeks. In fact, large pharmaceutical companies and research institutes around the world are accelerating the research and development of therapeutic antibodies that specifically bind to cancer cells, including cancer-causing factors, to effectively remove the cancer cells. Companies for developing therapeutic antibody drugs mainly consist of pharmaceutical companies, such as Roche, Amgen, Johnson & Johnson, Abbott, and BMS. Particularly, Roche includes representative products of Herceptin, Avastin, Rituxan, and the like for anticancer treatment, and not only produces large profits, achieving sales of approximately $19.5 billion in the global market in 2012 with these three therapeutic antibodies, but also leads the global antibody drug market. Johnson & Johnson, which developed Remicade, is also growing rapidly in the global antibody market due to increased sales, and pharmaceutical companies such as Abbott and BMS are also known to have many therapeutic antibodies in the final stages of development. As a result, in the global pharmaceutical market, where low molecular drugs had been dominant, the low molecular drugs have been rapidly replaced with biopharmaceuticals including therapeutic antibodies that are specific to disease targets and have low side effects. The antibody provides a link between the humoral and cellular immune systems, and a Fab region of the antibody recognizes an antigen, whereas an Fc domain part binds to a receptor (Fc receptor or FcR) for an antibody (immunoglobulin) on a cell that is differentially expressed by all immune competent cells and has a different mechanism depending on a type of FcγR expressed on the surface of the binding immune cell. An Fc receptor binding site on the Fc region of the antibody binds to the Fc receptor (FcR) on the cell, and the antibody binds to the Fc receptor on the cell surface through the Fc region to trigger a variety of important biological responses, including phagocy and destruction of antibody-coated particles, removal of immune complexes, lysis of antibody-coated target cells by killing cells (antibody-dependent cell-mediated cytotoxicity, or ADCC), release of inflammatory mediators, placental transfer and control of immunoglobulin production (Deo, Y.M. et al., Immunol. Today 18(3):127-135 (1997)). As such, the Fc domain plays a critical role in the recruitment of immune cells, and antibody-dependent cell-mediated cytotoxicity (ADCC) and antibody-dependent cell-mediated phagocytosis (ADCP). In particular, the ADCC and ADCP functions, which are effector functions of the antibody, depend on interaction with Fc receptors present on the surfaces of many cells. Human Fc receptors are classified into five types, and the type of immune cell to be recruited is determined depending on which Fc receptor the antibody binds to. For example, the Fc domain of the antibody is responsible for the major therapeutic effects of therapeutic antibodies by inducing the effector functions of ADCC by binding to FcγRIIIa, ADCP by binding to FcγRI or FcγRIIa, and complement dependent cytotoxicity (CDC) by binding to C1q to have toxicity as the target antigen binding to the Fab region.

However, in the therapeutic context, the effector functions of the antibody are often not preferred and may cause a safety problem and unwanted side effects by activating host immune defenses. For example, some therapeutic antibodies, such as immune checkpoint inhibitors that bind to immune cells and bispecific immune cell engagers, had a problem of side effects in which the immune mechanism is shown in the targeted immune cells to destroy the immune cells. Immune checkpoint inhibitors that target immune checkpoint proteins expressed on the surface of immune cells such as T cells have the disadvantage of lowering the original effects of antibodies by causing the side effects of destroying immune cells required to remove cancer cells by activating the immune responses due to an Fc-mediated immune mechanism. In addition, it has been reported that an immune cell activation inhibitory receptor (FcyRIIb) among FcyRs is expressed on T cells and reduces the efficacy of immune checkpoint inhibitory antibodies by interacting with the antibody Fc (Bennion et al., Sci Transl Med., 2023), and it is known that the interaction between the immune cell surface and the antibody Fc of FcγR is involved not only in the occurrence of side effects of immune checkpoint antibodies, but also in the reduction of efficacy. In addition, in a bispecific immune cell engager which is an antibody therapeutic agent acting to more effectively remove cancer cells by guiding immune cells to cancer cells, as an antibody therapeutic agent of which one side binds to an antigen of the surface of the cancer cell and the other side binds to the immune cell, when the corresponding antibody has an Fc-mediated immune mechanism, the immune cells are destroyed and the cancer cells are not effectively removed, resulting in side effects. In addition, there is a problem that agonist antibodies that bind to target cells to induce cell activation, or antagonist antibodies that block the interaction between the target antigen and the ligand, have toxicity to target cells and antigens due to the Fc-mediated immune mechanism, thereby reducing the original effects of the antibodies. In addition, when developing an Fc-fusion protein fused with the Fc region to increase the half-life of active substances such as proteins or chemicals for therapeutic, diagnostic, or research purposes, there is a problem of toxicity due to the Fc-mediated immune mechanism.

Therefore, in order to prevent off-target toxicity of an antibody due to the immune mechanism of the antibody and have an effective antibody therapeutic effect, it is essential to remove the Fc-mediated immune mechanism. To this end, when developing antibodies, IgG2 antibodies, which have a very low immune mechanism due to the lowest binding affinity to FcγR among human IgG subclasses, are considered. However, the IgG2 antibodies have various isotypes due to disulfide bond exchange in the hinge region, and have physical property problems that cause aggregation due to decreased stability, and thus IgG4 antibodies having the next lowest binding affinity are considered and have been currently used in clinical development. In this regard, anti-PD-1 antibodies (pembrolizumab (Keytruda) from Merck & Co., nivolumab (Opdivo) from Bristol-Myers Squibb, and cemiplimab (Libtayo) from Regeneron) targeting programmed cell death-1 (PD-1) as an immune checkpoint protein expressed on T cells have received FDA approval as all human IgG4 antibody forms and have been used in clinical trials in high demand. The pembrolizumab has received clinical approval for various types of cancer and ranked second in global pharmaceutical sales in 2020, with sales of $14.3 billion, and the nivolumab ranked eighth, with sales of $7.9 billion. However, the IgG4 antibodies also have binding affinity to all FcyRs, and particularly, have several nM of strong binding affinity to FcγRI, and thus have a problem of activating various immune mechanisms. Therefore, in order to prevent target cells from being destroyed by the immune mechanism of the antibodies, research has been actively conducted to produce Fc in which binding affinity to FcyRs is removed. In addition, wild-type human IgG4 had serine as an amino acid at position 228 of a CH₂ domain, unlike human IgG1, and an intrachain disulfide bond was formed through a flexible core hinge to produce a half-antibody with a noncovalent linkage. IgG4, which is present in such a half-antibody form, shows a Fab-arm exchange (FAE) phenomenon, in which two half-antibody forms of IgG4 targeting different antigens are combined. To prevent the phenomenon, if an amino acid at position 228 of human IgG4 is substituted with proline, which is an amino acid at position 228 of human IgG1, the hinge region is stabilized and Fab-arm exchange does not occur, and thus the corresponding S228P variant has been developed for general application to IgG4 clinical antibodies. However, the conventional S228P variant has the same FcyRs binding affinity as wild-type IgG4, and still has a problem of causing side effects of immune cell death/off-target toxicity to normal cells.

### [Disclosure]

### [Technical Problem]

An object of the present disclosure is to provide a novel human antibody Fc domain variant.

Another object of the present disclosure is to provide an antibody or fragment thereof or immunologically active fragment thereof having reduced effector functions.

Yet another object of the present disclosure is to provide an Fc-fusion protein.

Still another object of the present disclosure is to provide an antibody therapeutic agent.

Still another object of the present disclosure is to provide a pharmaceutical composition for preventing or treating cancer.

Still another object of the present disclosure is to provide a method for preparing a human antibody Fc domain variant.

Still another object of the present disclosure is to provide a method for preparing an antibody or fragment thereof having reduced effector functions.

Still another object of the present disclosure is to provide a use for the preparation of an antibody therapeutic agent.

Still another object of the present disclosure is to provide a use for preventing or treating cancer.

Still another object of the present disclosure is to provide a method for treating cancer.

### [Technical Solution]

In order to solve the problems, an aspect of the present disclosure provides a novel human antibody Fc domain variant having reduced effector functions.

Another aspect of the present disclosure provides an antibody or immunologically active fragment thereof including the novel human antibody Fc domain variant.

Yet another aspect of the present disclosure provides an Fc-fusion protein in which the human antibody Fc domain variant is fused with a protein therapeutic agent.

Still another aspect of the present disclosure provides an antibody therapeutic agent including the antibody or immunologically active fragment thereof, and a drug moiety.

Still another aspect of the present disclosure provides a pharmaceutical composition for preventing or treating cancer including the human antibody Fc domain variant, the antibody or immunologically active fragment thereof, or the antibody therapeutic agent as an active ingredient.

Still another aspect of the present disclosure provides a method for preparing the human antibody Fc domain variant.

Still another aspect of the present disclosure provides a method for preparing an antibody or fragment thereof having reduced effector functions.

Still another aspect of the present disclosure provides a use of an antibody or immunologically active fragment thereof including an Fc domain variant of the present disclosure for use in the preparation of an antibody therapeutic agent.

Still another aspect of the present disclosure provides a use of a human antibody Fc domain variant of the present disclosure, an antibody or immunologically active fragment thereof of the present disclosure, or an antibody therapeutic agent of the present disclosure for the prevention or treatment of cancer.

Still another aspect of the present disclosure provides a method for treating cancer including administering a human antibody Fc domain variant of the present disclosure, an antibody or immunologically active fragment thereof of the present disclosure, or an antibody therapeutic agent of the present disclosure in a pharmaceutically effective amount to a subject suffering from cancer.

### [Advantageous Effects]

According to the present disclosure, the human antibody Fc domain variants are novel variants that are different from conventional Fab-arm exchange-preventing variants, and can overcome the Fab-arm exchange phenomenon, which is a disadvantage of IgG4, while not binding to all human FcyRs and C1q and not binding to mouse and monkey FcyRs, and have excellent blood half-life and thermal stability, and thus can be used for the prevention of immune cell/normal cell death (toxicity) caused by an antibody Fc region of a therapeutic antibody or Fc-fusion protein drug.

### [Description of Drawings]

FIG. 1 is a diagram illustrating results of SDS-PAGE analysis after purification of Fab-arm exchange-preventing glycosylated IgG4 Fc variants (Stapled Fc-1, Stapled Fc-2, Stapled Fc-3, Stapled Fc-4, and Stapled Fc-5) in which binding affinity to FcyRs and C1q is removed.
FIG. 2 is a diagram illustrating results of SDS-PAGE analysis after expression and purification of purified FcyRI-GST, FcyRIIa-131H-GST, FcyRIIa-131R-GST, FcγRIIb-GST, FcγRIIIa-158V-GST and FcyRIIIa-158F-GST.
FIG. 3 is a diagram illustrating results of ELISA analysis for binding affinity of glycosylated pembrolizumab IgG4 Fc variants of the present disclosure to FcγRI, FcγRIIa-131H, FcyRIIa-131R, FcγRIIb, FcyRIIIa-158V, and FcyRIIIa-158F.
FIG. 4 is a diagram illustrating results of SDS-PAGE analysis after expression and purification of purified mouse FcyRI-GST, mouse FcyRIIb-GST, mouse FcγRIII-GST, and mouse FcγRIV-GST.
FIG. 5 is a diagram illustrating results of ELISA analysis for binding affinity of glycosylated pembrolizumab IgG4 Fc variants of the present disclosure to mouse FcγRI, mouse FcγRIIb, mouse FcγRIII, and mouse FcγRIV.
FIG. 6 is a diagram illustrating results of SDS-PAGE analysis after expression and purification of purified cynomolgus monkey FcyRI-GST, cynomolgus monkey FcγRIIa-GST, cynomolgus monkey FcγRIIb-GST, and cynomolgus monkey FcyRIII-GST.
FIG. 7 is a diagram illustrating ELISA analysis for binding affinity of glycosylated pembrolizumab IgG4 Fc variants of the present disclosure to cynomolgus monkey FcγRI, cynomolgus monkey FcγRIIa, cynomolgus monkey FcγRIIb, and cynomolgus monkey FcγRIII.
FIG. 8 is a diagram illustrating ELISA analysis for C1q binding affinity of glycosylated pembrolizumab IgG4 Fc variants of the present disclosure.
FIG. 9 is a diagram illustrating results of SDS-PAGE analysis after expression and purification of purified FcRn-GST.
FIG. 10 is a diagram illustrating ELISA analysis for pH-dependent FcRn binding affinity of glycosylated pembrolizumab IgG4 Fc variants of the present disclosure.
FIG. 11 is a diagram illustrating *in vivo* analysis for blood half-life of glycosylated pembrolizumab IgG4 Fc variants of the present disclosure in human FcRn expressing mice.
FIG. 12 is a diagram illustrating DSF analysis for thermal stability of glycosylated pembrolizumab IgG4 Fc variants of the present disclosure.

### [Best Mode]

Hereinafter, embodiments of the present disclosure will be described in detail with reference to the accompanying drawings. However, the following embodiments are presented as examples for the present disclosure, and when it is determined that the detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used herein are terminologies used to properly express preferred embodiments of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be meant to further comprise other components rather than excluding other components.

All technical terms used in the present disclosure, unless otherwise defined, are used in the meaning as commonly understood by those skilled in the related art of the present disclosure. In addition, although preferred methods and samples are described herein, similar or equivalent methods and samples thereto are also included in the scope of the present disclosure. The contents of all publications described herein as references are incorporated in the present disclosure.

Throughout the present specification, general one-letter or three-letter codes for naturally occurring amino acids are used, and generally allowed three-letter codes for other amino acids, such as α-aminoisobutyric acid (Aib) and N-methylglycine (Sar) are also used. The amino acids mentioned herein as abbreviations are also described as follows according to the IUPAC-IUB nomenclature.

Alanine: A; Arginine: R; Asparagine: N; Aspartic acid: D; Cysteine: C; Glutamic acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y; and Valine: V.

In one aspect, the present disclosure relates to a human antibody Fc domain variant in which any one or more amino acids selected from the group consisting of amino acids at positions 231, 232, 234, and 235 numbered according to a Kabat numbering system in a wild-type human antibody Fc domain are substituted with sequences different from wild-type amino acids.

In an embodiment, the human antibody Fc domain variant of the present disclosure may include any one or more amino acid substitutions selected from the group consisting of A231C, P232C, F234C, and L235C.

In an embodiment, the human antibody Fc domain variant of the present disclosure may be a human antibody Fc domain variant Stapled Fc-1 including an amino acid substitution of A231C, and the variant may include an amino acid sequence represented by SEQ ID NO: 1 and may be encoded by a nucleic acid molecule including a nucleotide sequence represented by SEQ ID NO: 2.

In an embodiment, the human antibody Fc domain variant of the present disclosure may be a human antibody Fc domain variant Stapled Fc-2 including an amino acid substitution of P232C, and the variant may include an amino acid sequence represented by SEQ ID NO: 3 and may be encoded by a nucleic acid molecule including a nucleotide sequence represented by SEQ ID NO: 4.

In an embodiment, the human antibody Fc domain variant of the present disclosure may be a human antibody Fc domain variant Stapled Fc-4 including an amino acid substitution of F234C, and the variant may include an amino acid sequence represented by SEQ ID NO: 5 and may be encoded by a nucleic acid molecule including a nucleotide sequence represented by SEQ ID NO: 6.

In an embodiment, the human antibody Fc domain variant of the present disclosure may be a human antibody Fc domain variant Stapled Fc-5 including an amino acid substitution of L235C, and the variant may include an amino acid sequence represented by SEQ ID NO: 7 and may be encoded by a nucleic acid molecule including a nucleotide sequence represented by SEQ ID NO: 8.

In an embodiment, the human antibody (immunoglobulin) may be IgA, IgM, IgE, IgD or IgG, or a variant thereof, more preferably IgG4.

In an embodiment, the human antibody (immunoglobulin) may be IgG4, or a variant thereof, may be a humanized antibody, and may be trastuzumab, pembrolizumab, or atezolizumab.

In an embodiment, the human antibody (immunoglobulin) may be IgG4 or a variant thereof, and the Fc domain of wild-type IgG4 including hinge, CH2 and CH3 may include an amino acid sequence represented by SEQ ID NO: 9 and may be encoded by a nucleic acid molecule including a nucleotide sequence represented by SEQ ID NO: 10.

In the present disclosure, the human antibody IgG4 represented by SEQ ID NO: 9 includes a Fc domain, i.e., hinge (amino acids at positions 1 to 12 of SEQ ID NO: 9), CH2 (amino acids at positions 13 to 122 of SEQ ID NO: 9), and CH3 (amino acids at positions 123 to 229 of SEQ ID NO: 9) in the entire sequence of wild-type IgG4, and the variants are variants in which the amino acids of CH2 of wild-type IgG4 are substituted. For example, in amino acids represented by SEQ ID NO: 9, an amino acid L at position 17 is an amino acid at position 235 numbered according to the Kabat numbering system, which is substituted with C in a Stapled Fc-5 (L235C) variant.

In an embodiment, the human antibody Fc domain variant of the present disclosure may have reduced binding affinity to Fc gamma receptors (FcyRs) compared to a wild-type human antibody Fc domain, and the Fc gamma receptors may be human, mouse or monkey Fc gamma receptors (FcγRs), and may be FcγRI, FcγRIIa, FcγRIIb, FcγRIII, FcγRIIIa or FcγRIV.

In an embodiment, the human FcyR may be FcγRI, FcγRIIa, FcγRIIb or FcγRIIIa, in which the FcγRIIa may be FcyRIIa-131H or FcyRIIa-131R; the FcγRIIIa may be FcγRIIIa-158V or FcγRIIIa-158F; the mouse FcyR may be mouse FcγRI, mouse FcγRIIb, mouse FcγRIII or mouse FcγIV; and the monkey FcyR may be cynomolgus monkey FcγRIIa, cynomolgus monkey FcγRIIb or monkey FcγRIII.

In an embodiment, the human antibody Fc domain variant of the present disclosure may have reduced binding affinity to C1q compared to the wild-type human antibody Fc domain.

In an embodiment, the human antibody Fc domain variant of the present disclosure may have reduced effector functions compared to the wild-type human antibody Fc domain.

In an embodiment, the effector function may be a Fc-mediated effector function selected from C1q-binding, complement activation, complement dependent cytotoxicity (CDC), antibody-dependent cellular cytotoxicity (ADCC), Fc-receptor binding including Fc-gamma receptor binding, protein A-binding, protein G-binding, antibody-dependent cell-mediated phagocytosis (ADCP), complement dependent cell-mediated cytotoxicity (CDCC), complement-enhanced cytotoxicity, opsonization, Fc-containing polypeptide internalization, target downmodulation, ADC uptake, induction of apoptosis, cell death, cell cycle arrest, and any combination thereof, preferably ADCC or CDC.

In an embodiment, the human antibody Fc domain variant of the present disclosure may have increased thermal stability compared to the wild-type human antibody Fc domain.

In an embodiment, the human antibody Fc domain variant of the present disclosure may have increased *in vivo* half-life compared to the wild-type human antibody Fc domain.

In an embodiment, the half-life of the human antibody Fc domain variant of the present disclosure may be increased at least 3%, at least 10%, at least 20%, at least 30%, at least 40%, at least 50%, at least 60%, at least 70%, at least 80%, at least 90%, or at least 100% compared to the wild-type human antibody Fc domain, or increased at least 2-fold, at least 3-fold, at least 4-fold, at least 5-fold, at least 6-fold, at least 7-fold, at least 8-fold, at least 9-fold, or at least 10-fold compared to the wild-type Fc domain.

In an embodiment, the human antibody Fc domain variant of the present disclosure may have high binding affinity to FcRn compared to the wild-type human antibody Fc domain at pH 5.6 to 6.5, and may be a weak acidic condition in the endosome, and pH 5.8 to 6.0. In the pH range, a pH-sensitive Fc variant of the present disclosure may have increased binding affinity to FcRn at least 10%, at least 20%, at least 30%, at least 40%, at least 50%, at least 60%, at least 70%, at least 80%, at least 90%, or at least 100% compared to the wild-type Fc domain, or increased at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 60-fold, at least 70-fold, at least 80-fold, at least 90-fold, or at least 100-fold compared to the wild-type Fc domain. In the embodiment, the Fc variant of the present disclosure may have low binding affinity to FcRn compared to a wild-type immunoglobulin Fc region at pH 7.0 to 7.8, may be a normal pH range of blood, and may be pH 7.2 to 7.6. The degree of dissociation of the Fc variant of the present disclosure from FcRn in the pH range may be the same or not substantially changed compared to the wild-type Fc domain.

In an embodiment, the Fc domain variants of the present disclosure may be used for the purpose of not killing binding cells.

In an embodiment, the Fc domain variants of the present disclosure may be applied to antibodies targeting immune cells or normal cells.

In an embodiment, the Fc domain variants of the present disclosure may be used in an immune checkpoint inhibitor antibody or a bispecific immune cell engaging bispecific antibody.

In the present disclosure, variants including amino acid mutations in the human antibody Fc domain of the present disclosure are defined according to amino acid modifications constituting an Fc domain of a parent antibody, and conventional antibody numbering is followed by the EU index according to Kabat (Kabat et al., Sequence of proteins of immunological interest, 5th Ed., United States Public Health Service, National Institutes of Health, Bethesda, 1991).

As used herein, the term "Fc domain variant" may be used interchangeably with the "Fc variant".

As used herein, the term "wild-type polypeptide" refers to an unmodified polypeptide that is modified later to produce a derivative. The wild-type polypeptide may be a polypeptide found in nature, or a derivative or manipulation of the polypeptide found in nature. The wild-type polypeptide may refer to a polypeptide itself, a composition including the wild-type polypeptide, or an amino acid sequence encoding the same. Accordingly, as used herein, the term "wild-type antibody" refers to an unmodified antibody polypeptide in which amino acid residues are modified to produce a derivative. Interchangeably with the term, the "parent antibody" may be used to refer to an unmodified antibody polypeptide into which amino acid modifications are introduced to produce a derivative.

As used herein, the term "amino acid modification/mutation" refers to substitution, insertion and/or deletion, preferably substitution of amino acids in a polypeptide sequence. As used herein, the term "amino acid substitution" or "substitution" means that an amino acid at a specific position in the polypeptide sequence of the wild-type human antibody Fc domain is replaced with another amino acid. For example, an Fc variant including an A231C substitution means that alanine, an amino acid residue at position 231 in the amino acid sequence of the wild-type antibody Fc domain, is replaced with cysteine.

As used herein, the term "Fc variant" means including a modification of one or more amino acid residues compared to the wild-type antibody Fc domain.

The Fc variant of the present disclosure includes one or more amino acid modifications compared to the wild-type antibody Fc domain (region or fragment), resulting in a difference in amino acid sequence. The amino acid sequence of the Fc variant according to the present disclosure is substantially the same as the amino acid sequence of the wild-type antibody Fc domain. For example, the amino acid sequence of the Fc variant according to the present disclosure has about 80% or more, preferably about 90% or more, and most preferably about 95% or more homology compared to the amino acid sequence of the wild-type antibody Fc domain. The amino acid modifications may be performed genetically using molecular biological methods, or also performed using enzymatic or chemical methods.

The Fc variant of the present disclosure may be prepared by any method known in the art. In an embodiment, the human antibody Fc variant according to the present disclosure is used to form a nucleic acid in which a polypeptide sequence including a specific amino acid modification is encoded and then, if desired, cloned into a host cell, expressed, and assayed. Various methods therefor are described in the literature (Molecular Cloning-A Laboratory Manual, 3rd Ed., Maniatis, Cold Spring Harbor Laboratory Press, New York, 2001; Current Protocols in Molecular Biology, John Wiley & Sons).

The nucleic acid encoding the Fc variant according to the present disclosure may be inserted into an expression vector for protein expression. The expression vector generally includes a control or regulatory sequence, a selectable marker, any fusion partner, and/or a protein operably linked, i.e., functionally related to an additional element. Under appropriate conditions, the Fc variant according to the present disclosure may be produced by a method for inducing the protein expression by incubating a host cell transformed with a nucleic acid, preferably a nucleic acid-containing expression vector encoding the Fc variant according to the present disclosure. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, the Fc variant according to the present disclosure is produced using *E. coli* having low production cost and high industrial value, as a host cell.

Accordingly, the scope of the present disclosure includes a method for preparing an Fc variant including incubating a host cell into which a nucleic acid encoding the Fc variant has been introduced, under conditions suitable for protein expression; and purifying or isolating the Fc variant expressed from the host cell.

As used herein, the term "FcRn" or "neonatal Fc receptor" refers to a protein that binds to an Fc domain of an IgG antibody, which is at least partially encoded by an FcRn gene. The FcRn may be derived from any organism, including humans, mice, rats, rabbits, and monkeys, but is not limited thereto. As known in the art, a functional FcRn protein includes two polypeptides, often referred to as light and heavy chains. The light chain is β-2-microglobulin, and the heavy chain is encoded by the FcRn gene. Unless otherwise stated herein, the FcRn or one FcRn protein refers to a complex of the FcRn heavy chain and the β-2-microglobulin.

In one aspect, the present disclosure relates to an antibody or immunologically active fragment thereof including the Fc domain variant of the present disclosure.

In an embodiment, the antibody or immunologically active fragment thereof may have reduced binding affinity to Fc gamma receptors (FcyRs) or C1q compared to a wild-type human antibody due to the removal of the binding affinity to FcyRs.

In an embodiment, the Fc gamma receptor may be human, mouse or monkey Fc gamma receptors (FcγRs), and may be FcγRI, FcγRIIa, FcγRIIb, FcγRIII, FcγRIIIa or FcγRIV.

In an embodiment, the human FcyR may be FcγRI, FcγRIIa, FcγRIIb or FcγRIIIa, in which the FcγRIIa may be FcyRIIa-131H or FcyRIIa-131R; the FcγRIIIa may be FcγRIIIa-158V or FcγRIIIa-158F; the mouse FcyR may be mouse FcγRI, mouse FcγRIIb, mouse FcγRIII or mouse FcγIV; and the monkey FcyR may be cynomolgus monkey FcγRIIa, cynomolgus monkey FcγRIIb or monkey FcγRIII.

In an embodiment, the antibody or immunologically active fragment thereof may have reduced effector functions compared to the wild-type human antibody.

In an embodiment, the antibody may be a polyclonal antibody, a monoclonal antibody, a minibody, a domain antibody, a bispecific antibody, an IgG-like bispecific antibody, a bispecific immune cell engager, an antibody mimetic, a chimeric antibody, an antibody conjugate, a human antibody, a humanized antibody, a bivalent antibody or a bispecific molecule. The immunologically active fragment may be Fab, Fd, Fab', dAb, F(ab'), F(ab')₂, a single chain fragment variable (scFv), Fv, a single chain antibody, an Fv dimer, a complementarity determining region fragment, or a diabody of the antibody.

The antibody may be isolated or purified by various methods known in the art. A standard purification method includes chromatography, electrophoresis, immunology, precipitation, dialysis, filtration, concentration, and chromatofocusing techniques. As known in the art, various natural proteins such as bacterial proteins A, G, and L bind to the antibody and these proteins may be used for purification. Often, purification by specific fusion partners may be enabled.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

The antibody or immunologically active fragment thereof of the present disclosure may be selected from the group consisting of animal-derived antibodies, chimeric antibodies, humanized antibodies, human antibodies, and immunologically active fragments thereof. The antibody may be produced recombinantly or synthetically.

The antibody or immunologically active fragment thereof may be isolated from a living body (not present in a living body) or may non-naturally occur, and for example, may be synthetically or recombinantly produced.

As used herein, the "antibody" refers to a material produced by stimulation of an antigen in the immune system, and the type thereof is not particularly limited, and may be obtained naturally or non-naturally (e.g., synthetically or recombinantly). The antibody is very stable *in vivo* as well as *ex vivo* and has long half-life, and thus is advantageous for mass expression and production. In addition, since the antibody essentially has a dimer structure, avidity is very high. An intact antibody has a structure having two full-length light chains and two full-length heavy chains, and each light chain is linked to the heavy chain via disulfide bonds. The constant region of the antibody is divided into a heavy chain constant region and a light chain constant region, and the heavy chain constant region has gamma (γ), mu (µ), alpha (α), delta (δ), and epsilon (ε) types, and has subclasses of gamma1 (γ1), gamma2 (γ2), gamma3 (γ3), gamma4 (γ4), alpha1 (α1), and alpha2 (α2). The light chain constant region has kappa (κ) and lambda (λ) types.

As used herein, the term "heavy chain" is interpreted as a meaning including all of a full-length heavy chain including a variable region domain V_{H} including an amino acid sequence having a sufficient variable region sequence to impart specificity to an antigen, three constant region domains C_{H}1, CH₂ and C_{H}3 and hinges, and fragments thereof. In addition, the term "light chain" is interpreted as a meaning including all of a full-length light chain including a variable region domain V_{L} including an amino acid sequence having a sufficient variable region sequence to impart specificity to an antigen and a constant region domain C_{L}, and fragments thereof.

As used herein, the term "Fc domain", "Fc fragment", or "Fc region" forms an antibody with a Fab domain/fragment, and the Fab domain/fragment consists of a light chain variable region V_{L} and a heavy chain variable region V_{H}, a light chain constant region C_{L}, and a first heavy chain constant region C_{H}1, and the Fc domain/fragment consists of a second constant region CH₂ and a third constant region C_{H}3 of the heavy chain.

In one aspect, the present disclosure relates to a nucleic acid molecule encoding the Fc domain variant of the present disclosure, or an antibody or immunologically active fragment thereof including the Fc domain variant.

In one aspect, the present disclosure relates to a vector including the nucleic acid molecule and a host cell including the vector.

The nucleic acid molecule of the present disclosure may be isolated or recombined, and includes not only DNA and RNA in single-stranded and double-stranded forms, but also complementary sequences corresponding thereto. The isolated nucleic acid is a nucleic acid that is isolated from surrounding genetic sequences present in the genome of a subject from which the nucleic acid is isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, DNA of a presequence or secretory leader is operably linked to DNA of the polypeptide when being expressed as a preprotein in the form before the polypeptide is secreted, a promoter or an enhancer is operably linked to a coding sequence when affecting the transcription of the polypeptide sequence, or a ribosome binding domain is operably linked to a coding sequence when disposed to promote the translation. In general, "operably linked" means that DNA sequences to be linked are located contiguously, and that the secretory leader exists contiguously in the same leading frame. However, the enhancer does not need to be contiguously located. The linkage is achieved by ligation at a convenient restriction enzyme site. When there is no such site, synthetic oligonucleotide adapters or linkers are used according to conventional methods.

In the isolated nucleic acid molecule encoding the Fc domain variant of the present disclosure, or the antibody or immunologically active fragment thereof including the Fc domain variant, due to the degeneracy of codons or in consideration of codons preferred in an organism in which the isolated nucleic acid molecule is to be expressed, it will be well understood by those skilled in the art that various modifications may be made to a coding region within a range without changing the amino acid sequence of the Fc domain variant, or the antibody or immunologically active fragment thereof including the Fc domain variant to be expressed from the coding region, various modifications or changes may be made within a range without affecting the expression of the gene even in parts other than the coding region, and such modified genes are also included within the scope of the present disclosure. That is, as long as the nucleic acid molecule of the present disclosure encodes a protein having equivalent activity thereto, one or more nucleotides may be mutated by substitution, deletion, insertion, or a combination thereof, which are also included in the scope of the present disclosure. The sequence of such a nucleic acid molecule may be single- or double-stranded, and may be a DNA molecule or an RNA (mRNA) molecule.

The isolated nucleic acid molecule encoding the Fc domain variant of the present disclosure, or the antibody or immunologically active fragment thereof including the Fc domain variant according to the present disclosure may be inserted into an expression vector for protein expression. The expression vector generally includes a control or regulatory sequence, a selectable marker, any fusion partner, and/or a protein operably linked, i.e., functionally related to an additional element. In appropriate conditions, the Fc domain variant of the present disclosure, or the antibody or immunologically active fragment thereof including the Fc domain variant may be produced by a method for inducing the protein expression by incubating a host cell transformed with a nucleic acid, preferably an expression vector containing an isolated nucleic acid molecule encoding the Fc domain variant of the present disclosure, the antibody or immunologically active fragment thereof including the Fc domain variant. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, it is possible to produce *E. coli*, which has high industrial value due to low production cost, as a host cell.

The vector of the present disclosure may include a plasmid vector, a cosmid vector, a bacteriophage vector, a viral vector, etc., but is not limited thereto. The suitable vector includes a signal sequence or a leader sequence for membrane targeting or secretion, in addition to expression regulatory elements such as a promoter, an operator, an initiation codon, a termination codon, a polyadenylation signal, and an enhancer, and may be variously produced according to the purpose. The promoter of the vector may be constitutive or inductive. The signal sequence may use a PhoA signal sequence, an OmpA signal sequence, etc. in the case of *Escherichia* sp. as a host, an α-amylase signal sequence, a subtilisin signal sequence, etc. in the case of *Bacillus* sp. as a host, an MFα signal sequence, an SUC2 signal sequence, etc. in the case of yeast as a host, and an insulin signal sequence, an α-FcγIV signal sequence, an antibody molecule signal sequence, etc. in the case of an animal cell as a host, but is not limited thereto. Further, the vector may include a selectable marker for selecting a host cell containing a vector and a replicable expression vector includes a replication origin.

As used herein, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (e.g., bacteriophages), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.).

In an embodiment, when constructing the vector, expression regulatory sequences such as a promoter, a terminator, and an enhancer, sequences for membrane targeting or secretion, etc. are appropriately selected according to a type of host cell to produce the Fc domain variant, or the antibody or immunologically active fragment thereof including the Fc domain variant, and may be variously combined depending on a purpose.

As used herein, the term "expression vector" refers to a vector including a nucleic acid sequence encoding at least a portion of a gene product to be transcribed. In some cases, an RNA molecule is then translated into a protein, a polypeptide, or a peptide. The expression vector may include various regulatory sequences. In addition to regulatory sequences that regulate transcription and translation, vectors and expression vectors may also include nucleic acid sequences that provide other functions.

As used herein, the term "host cell" includes eukaryotes and prokaryotes, and refers to any transformable organism capable of replicating the vector or expressing a gene encoded by the vector. The host cell may be transfected or transformed by the vector, which means a process in which an exogenous nucleic acid molecule is delivered or introduced into the host cell.

In an embodiment, the host cell may be bacteria or animal cells, the animal cell line may be a CHO cell, an HEK cell or an NSO cell, and the bacteria may be *E. coli.*

In one aspect, the present disclosure relates to a fusion protein in which a human Fc domain variant of the present disclosure, or an antibody or immunologically active fragment thereof is linked to a cargo molecule.

In an embodiment, the cargo molecule may be a detector, a therapeutic agent, a drug, a peptide, a growth factor, a cytokine, a receptor trap, a chemical compound, a carbohydrate moiety, an enzyme, an antibody or a fragment thereof, a DNA-based molecule, a viral vector, or a cytotoxic agent; at least one liposome or nanocarrier loaded with a detector, a therapeutic agent, a drug, a peptide, an enzyme, an antibody or a fragment thereof, a DNA-based molecule, a viral vector, or a cytotoxic agent; or one or more nanoparticles, nanowires, nanotubes, or quantum dots.

In an embodiment, the fusion protein may be an agonist antibody, an antagonist antibody or an antibody therapeutic agent.

In one aspect, the present disclosure relates to an Fc-fusion protein in which a human antibody Fc domain variant of the present disclosure is fused with a protein therapeutic agent.

In an embodiment, the protein therapeutic agent may be a T-cell modulatory polypeptide (TMP), an immune checkpoint protein or immune effector cell-specific targeting molecule, an immune checkpoint inhibitor antibody, a bispecific immune cell engaging bispecific antibody, an agonist antibody, or an antagonist antibody.

In an embodiment, the immune effector cell may be an effector T cell, a regulatory T cell, a natural killer (NK) cell, a natural killer T (NKT) cell, a dendritic cell, or a B cell.

In an embodiment, the immune checkpoint protein may be CD27, CD28, CD40, CD122, CD96, CD73, CD47, OX40, GITR, CSF1R, JAK, PI3K delta, PI3K gamma, TAM, arginase, CD137, ICOS, A2AR, B7-H3, B7-H4, BTLA, CTLA-4, LAG3, TIM3, VISTA, CD96, TIGIT, CD122, PD-1, PD-L1, or PD-L2.

In an embodiment, the immune checkpoint inhibitor antibody may be atezolizumab, avelumab, durvalumab, ipilimumab, IPH4102, IPH43, IPH33, lirimumab, monalizumab, nivolumab, pembrolizumab, and derivatives or functional equivalents thereof.

In one aspect, the present disclosure relates to an antibody therapeutic agent including an antibody or immunologically active fragment thereof of the present disclosure, and a drug moiety.

In an embodiment, the drug moiety may be an immunomodulatory drug (IMiD), an immunogenic apoptosis inducer, a microtubulin structure formation inhibitor, a meiosis inhibitor, a topoisomerase inhibitor, a DNA intercalator, a toxin, a chimeric antigen receptor (CAR) cell therapy, an oncolytic drug, an immunotherapy agent, a cytotoxic agent, an angiogenesis inhibitor, a kinase inhibitor, a costimulatory molecule blocker, an adhesion molecule blocker, an anti-cytokine agent, an anti-CTLA-4 agent, an anti-PD-1 agent, an anti-PD-L1 agent, an anti-PD-L2 agent, a TNF-α cross-linking agent, a TRAIL cross-linking agent, an anti-CD27 agent, an anti-CD30 agent, an anti-CD40 agent, an anti-4-1BB agent, an anti-GITR agent, an anti-OX40 agent, an anti-TRAILR1 agent, an anti-TRAILR2 agent, tagretin, FcγIV-alpha, clobetasol, peg FcγIV, prednisone, romidepsin, bexarotene, methotrexate, triamcinolone cream, anti-chemokine, vorinostat, Gabapentin, cyclosporine, rapamycin, FK506, a detectable marker or reporter, a TNF antagonist, an antirheumatic agent, a muscle relaxant, narcotic, a non-steroidal anti-inflammatory drug (NSAID), analgesic, anesthetic, sedative, local anesthetic, neuromuscular blocker, antibacterial, psoriasis therapeutic agent, corticosteroid, anabolic steroid, erythropoietin, immunization, immunoglobulin, immunosuppressant, growth hormone, hormone replacement drug, radiopharmaceutical, antidepressant, antipsychotic, stimulant, asthma drug, beta agonist, inhaled steroid, epinephrine or analogue thereof, cytokine, cytokine antagonist, PD-1 antagonist, adenosine A2AR antagonist, CD73 inhibitor, CTLA-4 inhibitor, TIM-3 inhibitor, LAG-3 inhibitor, anthracycline, or any combinations thereof.

In an embodiment, the antibody therapeutic agent may have reduced effector functions.

In an embodiment, the antibody therapeutic agent may be an immune checkpoint inhibitor or a bispecific immune cell engager.

In an aspect, the present disclosure relates to a pharmaceutical composition for preventing or treating cancer, including a human antibody Fc domain variant of the present disclosure, an antibody or immunologically active fragment thereof including the same, an Fc-fusion protein or an antibody therapeutic agent as an active ingredient.

In an embodiment, the cancer may be any one selected from the group consisting of brain tumor, melanoma, myeloma, non-small cell lung cancer, oral cancer, liver cancer, gastric cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

In an embodiment, the composition of the present disclosure may further include an immunogenic apoptosis inducing agent. The immunogenic apoptosis inducing agent may be any one or more selected from the group consisting of anthracycline-based anticancer agents, taxane-based anticancer agents, anti-EGFR antibodies, BK channel agonists, bortezomib, cardiac glycoside, cyclophosmide-based anticancer agents, GADD34/PP1 inhibitors, LV-tSMAC, Measles virus, bleomycin, mitoxantrone, or oxaliplatin. The anthracycline-based anticancer agent may be daunorubicin, doxorubicin, epirubicin, idarubicin, pixantrone, sabarubicin, or valrubicin, and the taxane-based anticancer agent may be paclitaxel or docetaxel.

The pharmaceutical composition of the present disclosure may be used as a single therapy, but may also be used in combination with other conventional biological therapies, chemotherapy or radiation therapy, and may treat more effectively cancer in the case of such concurrent therapy.

The pharmaceutical composition for preventing or treating cancer of the present disclosure is administered together with a chemical anticancer drug (anticancer agent) and the like to increase a cancer treatment effect of conventional anticancer agents through a cancer cell killing effect. Combined administration may be performed simultaneously or sequentially with the anticancer agent. Examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics such as dactinomycin (actinomycin D), plicamycin, and mitomycin C; plant alkaloids such as vincristine, vinblastine, etoposide, teniposide, topotecan, and iridotecan; and the like, but are not limited thereto.

As used herein, the term "prevention" means all actions that inhibit or delay the occurrence, spread, and recurrence of cancer by administration of the pharmaceutical composition according to the present disclosure.

As used herein, the term "treatment" refers to all actions that improve or beneficially change the death of cancer cells or the symptoms of cancer by administration of the composition of the present disclosure. Those skilled in the art to which the present disclosure pertains will be able to determine the degree of improvement, enhancement and treatment by knowing the exact criteria of disease for which the composition of the present disclosure is effective by referring to data presented by the Korean Medical Association, etc.

As used herein, the term "therapeutically effective amount" used in combination with the active ingredient means an amount of pharmaceutically acceptable salt of the composition effective for preventing or treating a target disease, and the therapeutically effective amount of the composition of the present disclosure may vary depending on many factors, such as a method of administration, a target site, the condition of a patient, and the like. Accordingly, when used in the human body, a dose should be determined as an appropriate amount in consideration of both safety and efficiency. It is also possible to estimate an amount to be used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. As used herein, the term "pharmaceutically effective amount" refers to an amount enough to treat the disease at a reasonable benefit/risk ratio applicable to medical treatment and enough not to cause side effects. The effective dose level may be determined according to factors including the health condition of a patient, the type and severity of cancer, the activity of a drug, the sensitivity to a drug, a method of administration, a time of administration, a route of administration, an excretion rate, duration of treatment, and drugs used in combination or simultaneously, and other factors well-known in the medical field. The composition of the present disclosure may be administered as an individual therapeutic agent or in combination with other therapeutic agents, and may be administered sequentially or simultaneously with conventional therapeutic agents, and may be administered at a single dose or in multiple doses. It is important to administer an amount capable of obtaining a maximum effect with a minimal amount without side effects by considering all the factors, which may be easily determined by those skilled in the art.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc and the like. The pharmaceutically acceptable additive according to the present disclosure is preferably included in an amount of 0.1 parts by weight to 90 parts by weight based on the composition, but is not limited thereto.

The composition of the present disclosure may include a carrier, a diluent, an excipient, or a combination of two or more thereof, which are commonly used in biological agents. The pharmaceutically acceptable carrier is not particularly limited as long as the carrier is suitable for *in vivo* delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be formulated in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be formulated preferably according to each disease or ingredient using a suitable method in the art or a method disclosed in Remington's Pharmaceutical Science (Mack Publishing Company, Easton PA, 18th, 1990).

The composition of the present disclosure may be administered parenterally (e.g., applied with injectable formulations intravenously, subcutaneously, intraperitoneally or topically) or orally according to a desired method, and the range of the dose may vary depending on the body weight, age, sex, and health condition of a patient, a diet, an administration time, an administration method, an excretion rate, the severity of a disease, and the like. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present disclosure relates to a method for preparing a human antibody Fc domain variant, including a) incubating a host cell including a vector including a nucleic acid molecule encoding the human antibody Fc domain variant of the present disclosure; and b) recovering a polypeptide expressed from the host cell.

In one aspect, the present disclosure relates to a method for preparing an antibody or fragment thereof having reduced effector functions, including a) incubating a host cell including a vector including a nucleic acid molecule encoding the antibody or immunologically active fragment thereof of the present disclosure; and b) purifying the antibody expressed from the host cell.

In an embodiment, the purification of the antibody may include filtration, HPLC, anion exchange or cation exchange, high performance liquid chromatography (HPLC), affinity chromatography, or a combination thereof, and preferably affinity chromatography using protein A.

In one aspect, the present disclosure provides a use of an antibody or immunologically active fragment thereof including an Fc domain variant of the present disclosure for use in the preparation of an antibody therapeutic agent.

In one aspect, the present disclosure provides a use of a human antibody Fc domain variant of the present disclosure, an antibody or immunologically active fragment thereof of the present disclosure, or an antibody therapeutic agent of the present disclosure for the prevention or treatment of cancer.

In one aspect, the present disclosure relates to a method for treating cancer including administering a human antibody Fc domain variant of the present disclosure, an antibody or immunologically active fragment thereof of the present disclosure, or an antibody therapeutic agent of the present disclosure in a pharmaceutically effective amount to a subject suffering from cancer.

### [Modes]

Hereinafter, the present disclosure will be described in more detail through the following Examples. However, the following Examples are only intended to embody the contents of the present disclosure, and the present disclosure is not limited thereto.

### Example 1. Production of Fab-arm exchange-preventing IgG4 Fc variants having removed FcγRs binding affinity

In order to produce Fc variants having removed binding affinity to FcyRs and C1q for preventing a Fab-arm exchange phenomenon in which half-antibody forms of wild-type human IgG4 were combined and avoiding a toxicity problem of a conventional S228P Fc variant, various Fab-arm exchange-preventing variants were produced by introducing cysteine into a lower hinge of a glycosylated Fc variant SL001 (E233C) having removed binding affinity to FcyRs in the previous study (Korean Patent Application No. 10-2023-0077425) of the present inventors (Table 1).

**[Table 1]**

| **Fc variants** | **Mutations** |
|---|---|
| IgG1 wild-type | - |
| IgG4 wild-type | - |
| IgG4-S228P | S228P |
| IgG4-SPLE | S228P/L235E |
| Stapled Fc-1 | A231C |
| Stapled Fc-2 | P232C |
| Stapled Fc-3 | E233C |
| Stapled Fc-4 | F234C |
| Stapled Fc-5 | L235C |

### Example 2. Expression and purification of glycosylated pembrolizumab IgG4 Fc variants including Fc variants

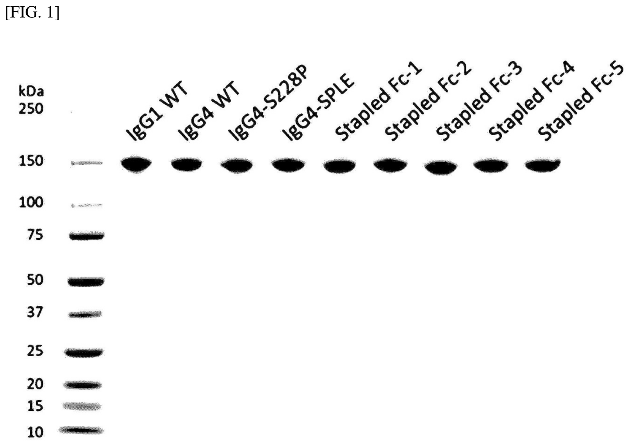

An expression vector was produced by cloning the glycosylated Fc variants produced in Example 1 above into a pembrolizumab heavy chain gene without a Fab-arm exchange variant as a model antibody. Thereafter, a heavy chain gene and a light chain gene of the variants were first mixed in 3 ml of a Freestyle 293 expression culture medium (Gibco, 12338-018) at a 1 : 1 ratio, and mixed at a ratio of PEI : variant gene = 4 : 1, left at room temperature for 20 minutes, and then mixed with Expi293F cells subcultured at the previous day with a density of 2 × 10⁶ cells/ml and incubated for 7 days under conditions of 37°C, 125 rpm, and 8% CO₂ and centrifuged to collect only the supernatant. The supernatant was equilibrated with 25X PBS and then filtered and prepared through a 0.2 µm syringe filter. Thereafter, protein A resin was added to the culture medium containing pembrolizumab Fc variants, stirred at 4°C for 16 hours, and then spun down to recover a resin, washed with 2 ml of PBS, and eluted with 600 µl of a 100 mM glycine (pH 2.7) buffer. The eluate was neutralized using 200 µl of 1 M Tris-HCl (pH 8.0) and the buffer was exchanged using Amicon Ultra-4 centrifugal filter units 30K (Merck Millipore, UFC503096), and then it was confirmed through SDS-PAGE gel analysis that the glycosylated antibody pembrolizumab Fc variants were purified with high purity. In particular, it was confirmed that the wild-type IgG4 antibody produced half-antibodies (75 kDa), whereas the glycosylated pembrolizumab Fc variants of the present disclosure all did not produce half-antibodies to prevent Fab-arm exchange (FIG. 1).

### Example 3. Analysis of binding affinity of glycosylated pembrolizumab IgG4 Fc variants to human FcγRs

### 3-1. Expression and purification of human FcγRs

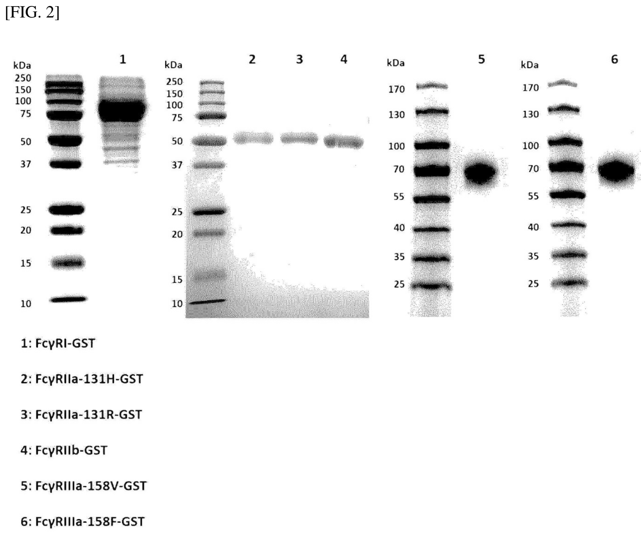

In order to analyze the binding affinity of the glycosylated Fc variants of the present disclosure to FcyRs using ELISA, FcyRI-GST, FcyRIIa-131H-GST, FcyRIIa-131R-GST, FcyRIIb-GST, FcyRIIIa-158V-GST, and FcyRIIIa-158F-GST were each cloned into an animal cell expression vector, transfected into Expi293F cells using PEI, and then incubated for 7 days under conditions of 37°C, 125 rpm, and 8% CO₂. After the incubation, the supernatant was collected, equilibrated with 25X PBS, and each receptor protein was purified using anti-GST affinity chromatography. Thereafter, SDS-PAGE analysis was performed to confirm that FcyRI-GST, FcγRIIa-131H-GST, FcyRIIa-131R-GST, FcyRIIb-GST, FcyRIIIa-158V-GST, and FcγRIIIa-158F-GST were purified with high purity (FIG. 2).

### 3-2. Analysis of binding affinity to FcγRs

To confirm FcyRs binding affinity of the glycosylated pembrolizumab Fc variants purified in Example 2 above, ELISA assay was performed. Specifically, FcγRs-GSTs (FcyRI-GST, FcγRIIa-131H-GST, FcyRIIa-131R-GST, FcyRIIb-GST, FcyRIIIa-158V-GST and FcyRIIIa-158F-GST) purified in Example 3-1 above were diluted to 4 µg/ml in 0.05 M Na₂CO₃ pH 9.6, 50 µl each of FcγRs-GSTs was immobilized on a flat bottom polystyrene high bind 96 well microplate (Costar, 3590) for 16 hours at 4°C, and then blocked with 100 µl of 4% skim milk (GenomicBase, SKI400) at room temperature for 1 hour. The microplate was washed four times with 180 µl of 0.05% PBST, and then 50 µl each of glycosylated pembrolizumab Fc variants serially diluted with 1% skim milk was dispensed into each well and reacted at room temperature for 1 hour. After washing, the microplate was subjected to antibody reaction at room temperature for 1 hour using 50 µl of HRP-Protein L (GenScript, M00098), and then washed again. The microplate was added with 50 µl each of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was analyzed using an Epoch microplate spectrophotometer (BioTek).

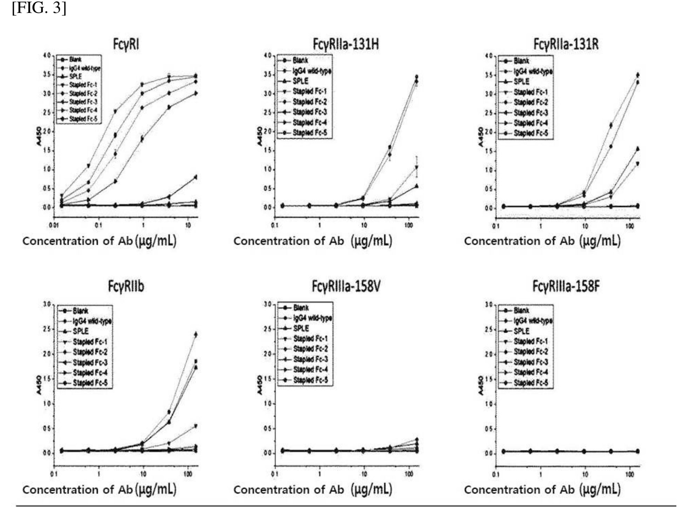

As a result, a Stapled Fc-1 (A231C) variant showed reduced binding affinity to all FcγRs except for FcγRI compared to a wild-type IgG4 antibody, a Stapled Fc-2 (P232C) variant showed similar binding affinity to FcyRs to the wild-type IgG4 antibody, and a Stapled Fc-4 (F234C) variant showed removed binding affinity to all FcyRs except for FcγRI (FIG. 3). In particular, a Stapled Fc-5 (L235C) variant showed removed binding affinity to all FcγRs and had significantly lower FcyRs binding affinity than a preceding S228P/L235E variant (SPLE), which prevented Fab-arm exchange and reduced FcyRs binding affinity in variants which have been used clinically in the related art (FIG. 3).

### Example 4. Analysis of binding affinity of glycosylated pembrolizumab IgG4 Fc variants to mouse FcγRs

### 4-1. Expression and purification of mouse FcγRs

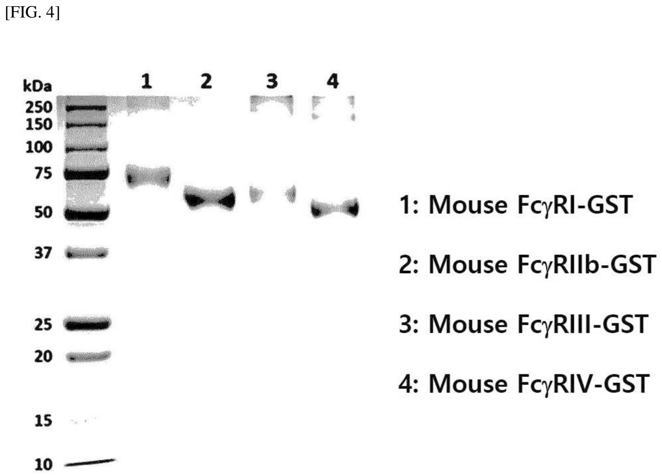

In order to analyze the binding affinity of the glycosylated Fc variants of the present disclosure to mouse FcyRs, which were widely used as preclinical animal models, using ELISA, mouse FcγRI-GST, mouse FcyRIIb-GST, mouse FcyRIII-GST, and mouse FcγRIV-GST were each cloned into an animal cell expression vector, transfected into Expi293F cells using PEI, and then incubated for 7 days under conditions of 37°C, 125 rpm, and 8% CO₂. After the incubation, the supernatant was collected, equilibrated with 1X PBS, and each receptor protein was purified using anti-GST affinity chromatography. Thereafter, SDS-PAGE analysis was performed to confirm that mouse FcγRI-GST, mouse FcyRIIb-GST, mouse FcyRIII-GST, and mouse FcyRIV-GST were purified with high purity (FIG. 4).

### 4-2. Analysis of binding affinity to mouse FcyRs

To confirm binding affinity of the glycosylated pembrolizumab Fc variants purified in Example 2 above to mouse FcγRs, ELISA assay was performed. Specifically, the mouse FcyRI-GST, mouse FcyRIIb-GST, mouse FcyRIII-GST, and mouse FcγRIV-GST purified in Example 4-1 above were diluted to 4 µg/ml in 0.05 M Na₂CO₃ pH 9.6 and 50 µl each was immobilized on a flat bottom polystyrene high bind 96 well microplate (Costar, 3590) for 16 hours at 4°C, and then blocked with 100 µl of 4% skim milk (GenomicBase, SKI400) at room temperature for 1 hour. The microplate was washed four times with 180 µl of 0.05% PBST, and then 50 µl each of glycosylated pembrolizumab Fc variants serially diluted with 1% skim milk was dispensed into each well and reacted at room temperature for 1 hour. After washing, the microplate was subjected to antibody reaction at room temperature for 1 hour using 50 µl of HRP-Protein L (GenScript, M00098), and then washed again. The microplate was added with 50 µl each of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was analyzed using an Epoch microplate spectrophotometer (BioTek).

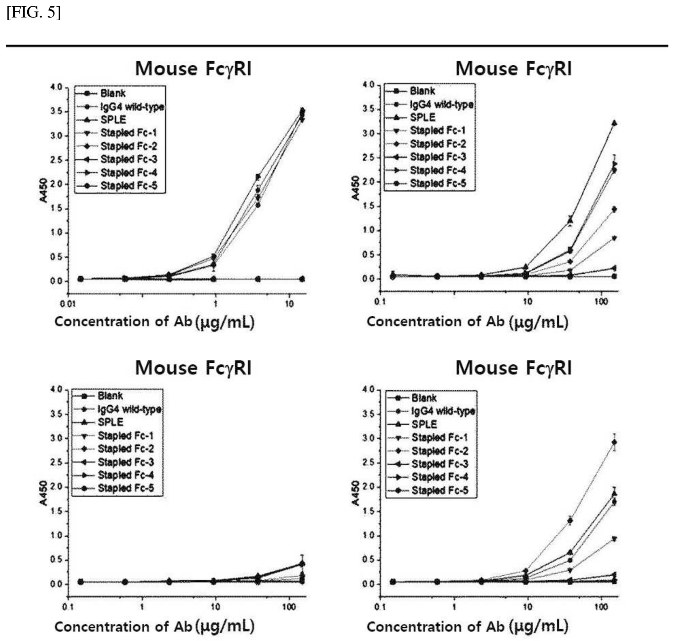

As a result, a Stapled Fc-5 (L235C) variant was found to have significantly lower FcγRs binding affinity than the wild-type IgG4 antibody and a conventional preceding S228P/L235E variant (SPLE), and particularly, the binding affinity to all mouse FcγRs was completely removed (FIG. 5).

### Example 5. Analysis of binding affinity of glycosylated pembrolizumab Fc variants to monkey FcγRs

### 5-1. Expression and purification of cynomolgus monkey FcγRs

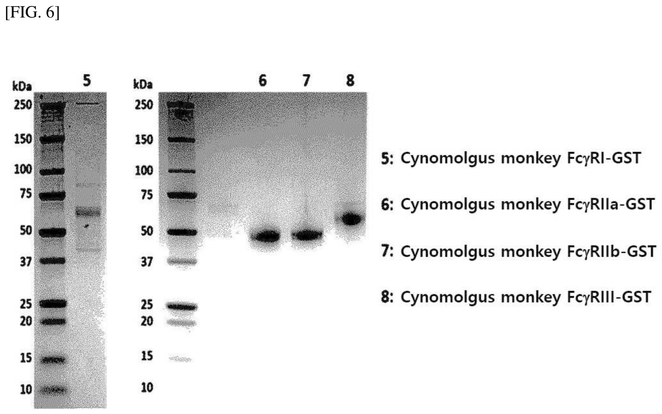

In order to analyze binding affinity of the glycosylated Fc variants of the present disclosure to cynomolgus monkey FcyRs, which were widely used as preclinical animal models, using ELISA, cynomolgus monkey FcyRI-GST, cynomolgus monkey FcyRIIa-GST, cynomolgus monkey FcγRIIb-GST, and cynomolgus monkey FcyRIII-GST were each cloned into an animal cell expression vector, transfected into Expi293F cells using PEI, and then incubated for 7 days under conditions of 37°C, 125 rpm, and 8% CO₂. After the incubation, the supernatant was collected, equilibrated with 25X PBS, and each receptor protein was purified using anti-GST affinity chromatography. Thereafter, SDS-PAGE analysis was performed to confirm that cynomolgus monkey FcγRI-GST, cynomolgus monkey FcyRIIa-GST, cynomolgus monkey FcγRIIb-GST, and cynomolgus monkey FcyRIII-GST were purified with high purity (FIG. 6).

### 5-2. Analysis of binding affinity to cynomolgus monkey FcγRs

To confirm binding affinity of the glycosylated pembrolizumab Fc variants purified in Example 2 above to cynomolgus monkey FcyRs, ELISA assay was performed. Specifically, the cynomolgus monkey FcγRI-GST, cynomolgus monkey FcyRIIa-GST, cynomolgus monkey FcγRIIb-GST, and cynomolgus monkey FcyRIII-GST purified in Example 5-1 above were diluted to 4 µg/ml in 0.05 M Na₂CO₃ pH 9.6 and 50 µl each was immobilized on a flat bottom polystyrene high bind 96 well microplate (Costar, 3590) for 16 hours at 4°C, and then blocked with 100 µl of 4% skim milk (GenomicBase, SKI400) at room temperature for 1 hour. The microplate was washed four times with 180 µl of 0.05% PBST, and then 50 µl each of glycosylated pembrolizumab Fc variants serially diluted with 1% skim milk was dispensed into each well and reacted at room temperature for 1 hour. After washing, the microplate was subjected to antibody reaction at room temperature for 1 hour using 50 µl of HRP-Protein L (GenScript, M00098), and then washed again. The microplate was added with 50 µl each of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was analyzed using an Epoch microplate spectrophotometer (BioTek).

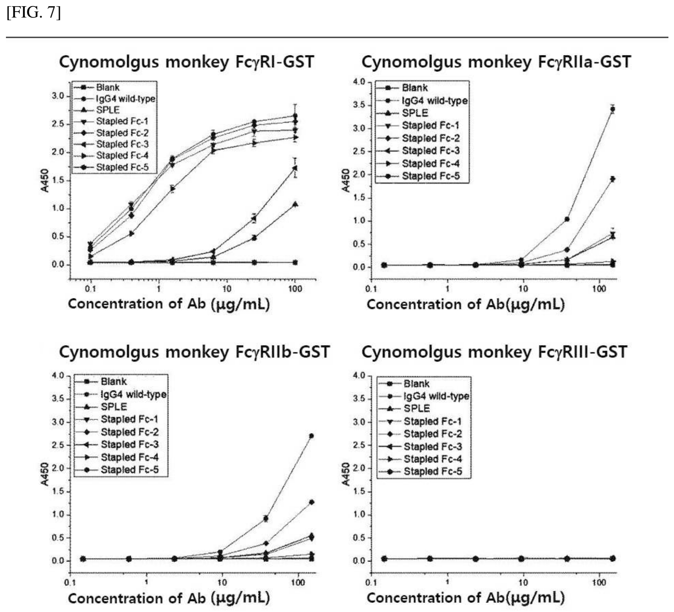

As a result, a Stapled Fc-5 (L235C) variant was found to have significantly lower FcγRs binding affinity than the wild-type IgG4 antibody and a conventional preceding S228P/L235E variant (SPLE), and particularly, the binding affinity to all cynomolgus monkey FcyRs was completely removed (FIG. 7).

### Example 6. Analysis of binding affinity of glycosylated pembrolizumab Fc variants to human C1q

To confirm C1q binding affinity of the glycosylated pembrolizumab Fc variants purified in Example 2 above, ELISA assay was performed. Specifically, 50 µl each of the glycosylated pembrolizumab Fc variants diluted to 4 µg/ml in 0.05 M Na₂CO₃ (pH 9.6) was immobilized on a flat bottom polystyrene high bind 96 well microplate (Costar, 3590) for 16 hours at 4°C, and then blocked with 100 µl of 4% skim milk (GenomicBase, SKI400) at room temperature for 1 hour. The microplate was washed four times with 180 µl of 0.05% PBST, and then 50 µl each of C1q (Quidel, A400) protein serially diluted with 1% skim milk was dispensed into each well and reacted at room temperature for 1 hour. After washing, the microplate was subjected to antibody reaction for 1 hour at room temperature using 50 µl of anti-C1q-HRP (Invitrogen, PA1-84324) and then washed again. The microplate was added with 50 µl of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was analyzed using an Epoch microplate spectrophotometer (BioTek).

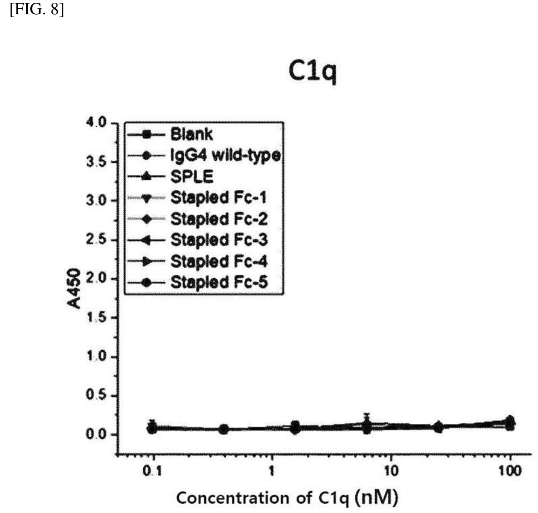

As a result, it was shown that all of the glycosylated pembrolizumab Fc variants of the present disclosure had no binding affinity to C1q (FIG. 8).

### Example 7. In vivo half-life analysis of glycosylated pembrolizumab Fc variants

### 7-1. Analysis of binding affinity to human FcRn

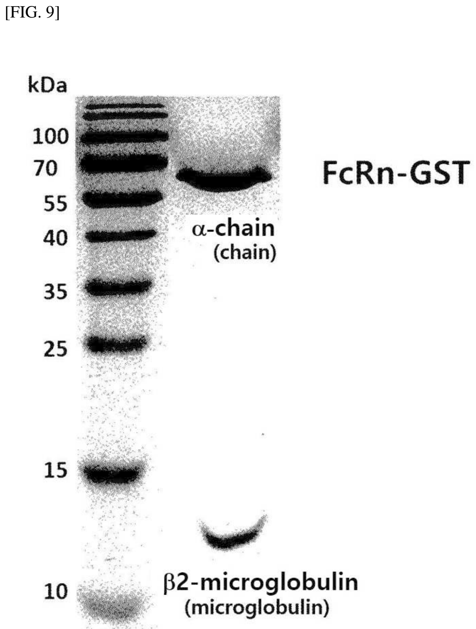

To confirm binding affinity to human FcRn, which was involved in the *in vivo* half-life of the glycosylated Fc variants of the present disclosure, ELISA assay was performed according to pH. First, FcRn-GST was cloned and prepared into an animal cell expression vector, transfected into Expi293F cells using PEI, and then incubated for 7 days under conditions of 37°C, 125 rpm, and 8% CO₂. After the incubation, the supernatant was recovered, equilibrated with 25X PBS, and FcRn-GST was purified using anti-GST affinity chromatography, and then high purity purification was confirmed by SDS-PAGE analysis (FIG. 9). In addition, the glycosylated pembrolizumab Fc variants purified in Example 2 above were diluted to 4 µg/ml in 0.05 M Na₂CO₃ pH 9.6, 50 µl each was immobilized on a flat bottom polystyrene high bind 96 well microplate (Costar, 3590) for 16 hours at 4°C, and then blocked with 100 µl of 4% skim milk (GenomicBase, SKI400) at room temperature for 1 hour. The microplate was washed 4 times with 180 µl of 0.05% PBST, and then the purified FcRn-GST was serially diluted with 1% skim milk (pH 6.0/pH 7.4) and 50 µl each was dispensed into each well and reacted at room temperature for 1 hour. After washing, 50 µl each of anti-GST-HRP conjugate (GE Healthcare, RPN1236V) was added to each well, and subjected to antibody reaction for 1 hour at room temperature, and then washed again. The microplate was added with 50 µl each of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was analyzed using an Epoch microplate spectrophotometer (BioTek).

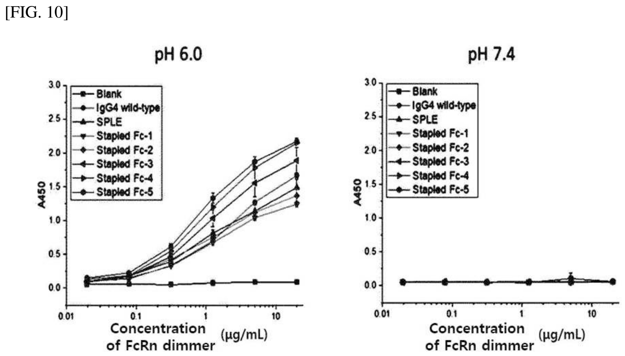

As a result, all of the glycosylated pembrolizumab Fc variants of the present disclosure were found to maintain the FcRn properties of the wild-type IgG4 antibody, and in particular, a Stapled Fc-5 (L235C) variant was found to have significantly improved binding affinity to FcRn at pH 6.0 compared to the wild-type IgG4 antibody (FIG. 10), confirming that the half-life was increased compared to the wild-type.

### 7-2. In vivo half-life analysis in human FcRn expressing mice

To compare and analyze *in vivo* half-life of the Stapled Fc-5 (L235C) variant, which was confirmed to have increased binding affinity to pH-dependent human FcRn, with that of a conventional variant S228P, pharmacokinetic analysis was performed using Tg276 female mice having human FcRn. Specifically, 2 or 3 mice per variant were each i.v. injected with pembrolizumab containing each variant at 5 mg/kg and then after 0.5, 24, 168, 336, 504, and 696 hours, blood was collected from each mouse and centrifuged at 1000 X g for 15 minutes to obtain the serum. To measure antibody concentrations in the serum using ELISA, 50 µl each of HER2-His diluted to 4 µg/ml in 0.05 M Na₂CO₃ pH 9.6 was immobilized on a flat bottom polystyrene high bind 96 well microplate (Costar, 3590) for 16 hours at 4°C, and then blocked with 100 µl of 4% skim milk (GenomicBase, SKI400) at room temperature for 1 hour. The microplate was washed four times with 180 µl of 0.05% PBST, and then pembrolizumab containing each variant was serially diluted from 1 µg/ml with 1% skim milk to make a standard curve, and 50 µl each of serum was dispensed into each well and reacted at room temperature for 1 hour. After washing, the microplate was subjected to antibody reaction at room temperature for 1 hour using 50 µl of goat anti-human IgG H+L (Jackson Immunoresearch, 109-036-003) and washed again. The microplate was added with 50 µl of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was analyzed using an Epoch microplate spectrophotometer (BioTek). Antibody concentrations were analyzed using a standard curve.

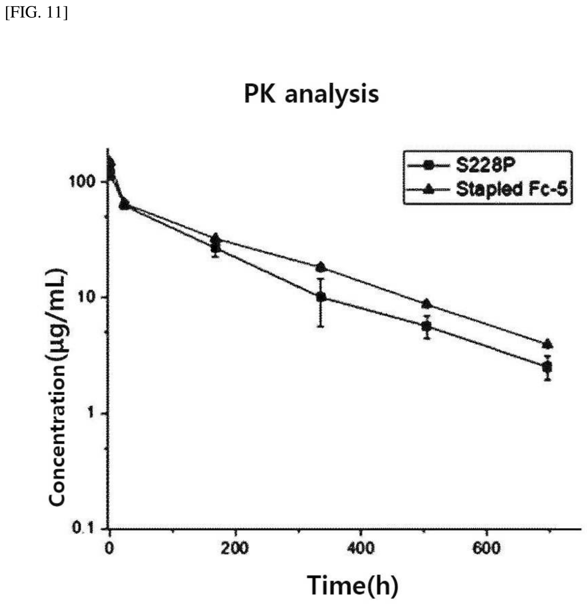

As a result, the Stapled Fc-5 (L235C) variant was found to have higher blood half-life than the S228P, which was a Fab-arm exchange-preventing variant that has been used clinically in the related art (FIG. 11).

### Example 8. Thermal stability analysis of glycosylated pembrolizumab Fc variants

To confirm thermal stability of the glycosylated pembrolizumab Fc variants purified in Example 2 above according to a temperature, differential scanning fluorimetry (DSF) analysis was performed. Specifically, 45 µl of the glycosylated pembrolizumab Fc variants diluted to 5 µM in 1X PBS and 5 µl of SYPRO Orange (Invitrogen, S6651) dye diluted to 200X were mixed and dispensed onto a PCR plate (Thermo Scientific, AB0900W), respectively, and 1X PBS was also prepared as a control in the same manner (all samples were performed in a triplicate). An optically clear sealing film (Thermo Scientific, AB1170) was attached to the plate containing the samples, and the fluorescence intensity was measured at a temperature increase of 0.03°C per second from 25°C to 99.9°C using a QuantStudio 3 Real-Time PCR System (Applied Biosystems, A28567). The fluorescence values according to each temperature were fitted to a Boltzmann model using OriginPro software, and then the midpoint of a sigmoidal transition curve was calculated.

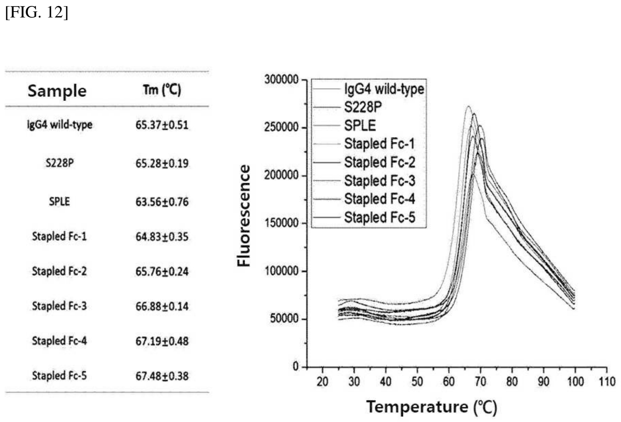

As a result, the glycosylated pembrolizumab Fc variants of the present disclosure were found to have higher degradation temperatures than the wild-type IgG4 antibody, the conventional Fab-arm exchange preventing variant (S228P), and the conventional preceding variant SPLE (S228P/L235E), and in particular, the Stapled Fc-5 (L235C) variant was found to have the highest degradation temperature (FIG. 12) to have excellent thermal stability.

## Claims

1. A human antibody Fc domain variant, wherein amino acids at any one or more positions selected from the group consisting of amino acids at positions 231, 232, 234, and 235 numbered according to a Kabat numbering system in a wild-type human antibody Fc domain are substituted with sequences different from wild-type amino acids.

2. The human antibody Fc domain variant of claim 1, wherein the human antibody Fc domain variant includes any one or more amino acid substitutions selected from the group consisting of A231C, P232C, F234C, and L235C.

3. The human antibody Fc domain variant of claim 1, wherein the human antibody is IgG4.

4. The human antibody Fc domain variant of claim 1, wherein binding affinity to Fc gamma receptors (FcγRs) is reduced compared to the wild-type human antibody Fc domain.

5. The human antibody Fc domain variant of claim 4, wherein the human antibody Fc domain variant is human, mouse or monkey Fc gamma receptors (FcyRs).

6. The human antibody Fc domain variant of claim 1, wherein binding affinity to C1q is reduced compared to the wild-type human antibody Fc domain.

7. The human antibody Fc domain variant of claim 1, wherein effector functions are reduced compared to the wild-type human antibody Fc domain.

8. The human antibody Fc domain variant of claim 7, wherein the effector function is an Fc-mediated effector function selected from C1q-binding, complement activation, complement dependent cytotoxicity (CDC), antibody-dependent cellular cytotoxicity (ADCC), Fc-receptor binding including Fc-gamma receptor binding, protein A-binding, protein G-binding, antibody-dependent cell-mediated phagocytosis (ADCP), complement dependent cell-mediated cytotoxicity (CDCC), complement-enhanced cytotoxicity, opsonization, Fc-containing polypeptide internalization, target downmodulation, ADC uptake, induction of apoptosis, cell death, cell cycle arrest, and any combination thereof.

9. The human antibody Fc domain variant of claim 1, wherein thermal stability is increased compared to the wild-type human antibody Fc domain.

10. The human antibody Fc domain variant of claim 1, wherein *in vivo* half-life is increased compared to the wild-type human antibody Fc domain.

11. An antibody or immunologically active fragment thereof, comprising the human antibody Fc domain variant of claim 1.

12. The antibody or immunologically active fragment thereof of claim 11, wherein binding affinity to Fc gamma receptors (FcγRs) is reduced compared to a wild-type human antibody.

13. The antibody or immunologically active fragment thereof of claim 11, wherein binding affinity to C1q is reduced compared to the wild-type human antibody.

14. The antibody or immunologically active fragment thereof of claim 11, wherein effector functions are reduced compared to the wild-type human antibody.

15. The antibody or immunologically active fragment thereof of claim 11, wherein the antibody is a polyclonal antibody, a monoclonal antibody, a minibody, a domain antibody, a bispecific antibody, an IgG-like bispecific antibody, a bispecific immune cell engager, an antibody mimetic, a chimeric antibody, an antibody conjugate, a human antibody, a humanized antibody, a bivalent antibody or a bispecific molecule.

16. A nucleic acid molecule encoding the human antibody Fc domain variant of claim 1, or the antibody or immunologically active fragment thereof of claim 11.

17. An Fc-fusion protein in which the human antibody Fc domain variant of claim 1 is fused with a protein therapeutic agent.

18. The Fc-fusion protein of claim 17, wherein the protein therapeutic agent is a T-cell modulatory polypeptide (TMP), an immune checkpoint protein or immune effector cell-specific targeting molecule, an immune checkpoint inhibitor antibody, a bispecific immune cell engaging bispecific antibody, an agonist antibody, or an antagonist antibody.

19. The Fc-fusion protein of claim 18, wherein the immune checkpoint protein is CD27, CD28, CD40, CD122, CD96, CD73, CD47, OX40, GITR, CSF1R, JAK, PI3K delta, PI3K gamma, TAM, arginase, CD137, ICOS, A2AR, B7-H3, B7-H4, BTLA, CTLA-4, LAG3, TIM3, VISTA, CD96, TIGIT, CD122, PD-1, PD-L1, or PD-L2.

20. The Fc-fusion protein of claim 18, wherein the immune checkpoint inhibitor antibody is atezolizumab, avelumab, durvalumab, ipilimumab, IPH4102, IPH43, IPH33, lirimumab, monalizumab, nivolumab, pembrolizumab, and derivatives or functional equivalents thereof.

21. An antibody therapeutic agent comprising the antibody or immunologically active fragment thereof of claim 11, and a drug moiety.

22. The antibody therapeutic agent of claim 21, wherein the drug moiety is an immunomodulatory drug (IMiD), an immunogenic apoptosis inducer, a microtubulin structure formation inhibitor, a meiosis inhibitor, a topoisomerase inhibitor, a DNA intercalator, a toxin, a chimeric antigen receptor (CAR) cell therapy, an oncolytic drug, an immunotherapy agent, a cytotoxic agent, an angiogenesis inhibitor, a kinase inhibitor, a costimulatory molecule blocker, an adhesion molecule blocker, an anti-cytokine agent, an anti-CTLA-4 agent, an anti-PD-1 agent, an anti-PD-L1 agent, an anti-PD-L2 agent, a TNF-α cross-linking agent, a TRAIL cross-linking agent, an anti-CD27 agent, an anti-CD30 agent, an anti-CD40 agent, an anti-4-1BB agent, an anti-GITR agent, an anti-OX40 agent, an anti-TRAILR1 agent, an anti-TRAILR2 agent, tagretin, interferon-alpha, clobetasol, peg interferon, prednisone, romidepsin, bexarotene, methotrexate, triamcinolone cream, anti-chemokine, vorinostat, Gabapentin, cyclosporine, rapamycin, FK506, a detectable marker or reporter, a TNF antagonist, an antirheumatic agent, a muscle relaxant, narcotic, a non-steroidal anti-inflammatory drug (NSAID), analgesic, anesthetic, sedative, local anesthetic, neuromuscular blocker, antibacterial, psoriasis therapeutic agent, corticosteroid, anabolic steroid, erythropoietin, immunization, immunoglobulin, immunosuppressant, growth hormone, hormone replacement drug, radiopharmaceutical, antidepressant, antipsychotic, stimulant, asthma drug, beta agonist, inhaled steroid, epinephrine or analogue thereof, cytokine, cytokine antagonist, PD-1 antagonist, adenosine A2AR antagonist, CD73 inhibitor, CTLA-4 inhibitor, TIM-3 inhibitor, LAG-3 inhibitor, anthracycline, or any combinations thereof.

23. The antibody therapeutic agent of claim 21, wherein the antibody therapeutic agent has reduced effector functions.

24. A pharmaceutical composition for preventing or treating cancer comprising the human antibody Fc domain variant of claim 1, the antibody or immunologically active fragment thereof of claim 11, the Fc-fusion protein of claim 17, or the antibody therapeutic agent of claim 21 as an active ingredient.

25. A method for preparing a human antibody Fc domain variant comprising:
a) incubating a host cell including a vector including a nucleic acid molecule encoding the human antibody Fc domain variant of claim 1; and
b) recovering a polypeptide expressed by the host cell.

26. A method for preparing an antibody or fragment thereof having reduced effector functions comprising:
a) incubating a host cell including a vector including a nucleic acid molecule encoding the antibody or immunologically active fragment thereof of claim 11; and
b) purifying the antibody expressed from the host cell.

27. A use of an antibody or immunologically active fragment thereof comprising the Fc domain variant of claim 1 for use in preparation of an antibody therapeutic agent.

28. A use of the human antibody Fc domain variant of claim 1, the antibody or immunologically active fragment thereof of claim 11, or the antibody therapeutic agent of claim 21 for prevention or treatment of cancer.

29. A method for treating cancer, comprising administering the human antibody Fc domain variant of claim 1, the antibody or immunologically active fragment thereof of claim 11, or the antibody therapeutic agent of claim 21 in a pharmaceutically effective amount to a subject suffering from cancer
